(19) Europäisches Patentamt / European Patent Office / Office européen des brevets

(11) **EP 2 959 908 A1**

(12) **EUROPEAN PATENT APPLICATION**
published in accordance with Art. 153(4) EPC

(43) Date of publication:
**30.12.2015 Bulletin 2015/53**

(21) Application number: **14754829.1**

(22) Date of filing: **24.02.2014**

(51) Int Cl.:
**A61K 33/44** (2006.01)     **A61K 31/785** (2006.01)
**A61P 1/16** (2006.01)      **A61P 13/12** (2006.01)
**A61P 39/02** (2006.01)

(86) International application number:
**PCT/JP2014/054265**

(87) International publication number:
**WO 2014/129618 (28.08.2014 Gazette 2014/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **22.02.2013   JP 2013033619**

(71) Applicant: **Kureha Corporation**
**Chuo-ku**
**Tokyo 103-8552 (JP)**

(72) Inventors:
• **WAKAHOI Takashi**
**Tokyo 103-8552 (JP)**
• **AKITA Takahiro**
**Tokyo 103-8552 (JP)**
• **SONOBE Naohiro**
**Tokyo 103-8552 (JP)**
• **KUWAHARA Mieko**
**Tokyo 103-8552 (JP)**

(74) Representative: **Zimmermann & Partner**
**Patentanwälte mbB**
**Postfach 330 920**
**80069 München (DE)**

(54) **ORALLY ADMINISTERED ADSORBENT, THERAPEUTIC AGENT FOR RENAL DISEASE, AND THERAPEUTIC AGENT FOR LIVER DISEASE**

(57)     An object of the present invention is to provide surface-modified spherical activated carbon exhibiting excellent adsorption capacity for uremic substances in the body, and particularly β-aminoisobutyric acid.

Accordingly, provided is an orally administered adsorbent comprising surface-modified spherical active carbon containing not less than 0.5 wt% of nitrogen atoms, having a specific surface area determined by the Brunauer-Emmett-Teller (BET) method of 800 $m^2$/g to 3000 $m^2$/g, having an average particle size of 0.01 mm to 1 mm, and having a total acidic group content of not less than 0.30 meq/g.

EP 2 959 908 A1

**Description**

TECHNICAL FIELD

The present invention relates to an orally administered adsorbent comprising surface-modified spherical activated carbon containing not less than 0.5 wt% nitrogen atoms. The present invention also relates to a therapeutic or prophylactic agent for a renal disease and a therapeutic or prophylactic agent for a hepatic disease that contain as an active ingredient the aforementioned orally administered adsorbent.
The orally administered adsorbent according to the present invention has excellent adsorption ability for uremic substances in the body, particularly β-aminoisobutyric acid.

BACKGROUND ART

Accompanying organ functional impairment in patients deficient in renal function or hepatic function, poisonous toxic substances accumulate and are produced in the body such as in the blood, and cause uremia or encephalopathy such as impaired consciousness. Since the number of such patients has shown an increasing trend year by year, the development of therapeutic medicines or organ substitute devices that have the function of removing toxic substances to outside the body in place of these deficient organs is a critical topic. Removal of poisonous substances by hemodialysis is currently the method most widely used as artificial kidneys. However, such hemodialysis-type artificial kidneys are not necessarily satisfactory due to problems such as the necessity for a specialized technician from the viewpoint of safety management because a special machine is used, and additionally, the high physical, mental and economic burden on the patient due to extracorporeal removal of blood, and the like.
As a means for solving these problems, an oral adsorbent that can be orally ingested and can treat functional impairment of the kidney or liver has been developed and used (Patent Document 1). The oral adsorbent has been widely clinically used in, for example, patients with hepatorenal functional impairment as an oral therapeutic agent that has less side effect such as constipation. The oral adsorbent contains a porous spherical carbonaceous substance (that is, spherical activated carbon) having a certain functional group, has excellent adsorbance of poisonous substances (that is, β-aminoisobutyric acid, γ-amino-n-butyric acid, dimethylamine, and octopamine) in the presence of bile acid in the intestines, and also has beneficial selective adsorbance in the sense that it adsorbs little of the beneficial components in the intestines such as digestive enzymes and the like. Furthermore, the adsorbent described in Patent Document 1 uses pitch such as petroleum pitch as a carbon source, and is produced by performing oxidation treatment and reduction treatment after preparation of the spherical activated carbon. The spherical activated carbon that has undergone this oxidation treatment and reduction treatment has been named surface-modified spherical activated carbon.
Additionally, Patent Document 2 discloses that surface-modified spherical activated carbon having an average particle size from 50 μm to 200 μm has excellent initial adsorption ability. That is, in the general residence time (within 3 hours) in the upper small intestine after ingestion of an orally administered adsorbent, it can very rapidly adsorb poisonous toxic substances (particularly β-aminoisobutyric acid) in the body.

CITATION LIST

Patent Literature

Patent Document 1: Japanese Examined Patent Application Publication No. S62-11611B
Patent Document 2: Japanese Unexamined Patent Application Publication No. 2005-314416A

SUMMARY OF INVENTION

Technical Problem

The above surface-modified spherical activated carbons described in Patent Documents 1 and 2 have excellent adsorption ability for uremic substances in the body, particularly β-aminoisobutyric acid. However, even the adsorption ability for uremic substances of the surface-modified spherical activated carbon described in Patent Documents 1 and 2 is insufficient, and further improvement has been anticipated.
An object of the present invention is to provide surface-modified spherical activated carbon exhibiting excellent adsorption ability with respect to uremic substances in the body, and particularly with respect to β-aminoisobutyric acid.

Solution to Problem

As a result of diligent research on surface-modified spherical activated carbon having excellent adsorption ability for uremic substances in the body, the present inventors unexpectedly discovered that surface-modified spherical activated carbon containing not less than 0.5 wt% nitrogen atoms exhibits excellent adsorption ability for uremic substances, particularly adsorption ability for β-aminoisobutyric acid. As the nitrogen atom quantity increases, the surface-modified spherical activated carbon has markedly increased adsorption ability for β-aminoisobutyric acid, and it is surprising that nitrogen atoms of surface-modified spherical activated carbon are related to adsorption ability for uremic substances.

The present invention is based on such knowledge.

Therefore, the present invention relates to the following.

[1] An orally administered adsorbent comprising surface-modified spherical activated carbon containing not less than 0.5 wt% nitrogen atoms, having specific surface area determined by the Brunauer-Emmett-Teller (BET) method from 800 $m^2/g$ to 3000 $m^2/g$, having an average particle size from 0.01 mm to 1 mm, and having total acidic group content of not less than 0.30 meq/g;

[2] The orally administered adsorbent according to [1], wherein the average particle size of the surface-modified spherical activated carbon is from 50 μm to 200 μm;

[3] The orally administered adsorbent according to [1] or [2], wherein the surface-modified spherical activated carbon has total acidic group content from 0.30 meq/g to 1.20 meq/g, and total basic group content from 0.20 meq/g to 1.20 meq/g;

[4] The orally administered adsorbent according to any one of [1] to [3], wherein the surface-modified spherical activated carbon is prepared using a thermoplastic resin, thermosetting resin, or ion exchange resin containing nitrogen atoms as a carbon source;

[5] The orally administered adsorbent according to [4], wherein the thermoplastic resin or ion exchange resin contains a monomer selected from the group consisting of acrylonitrile, ethylacrylonitrile, methylacrylonitrile, diphenylacrylonitrile, and chloroacrylonitrile;

[6] The orally administered adsorbent according to [4], wherein the thermosetting resin contains a monomer selected from the group consisting of melamine and urea;

[7] A therapeutic or prophylactic agent for a renal disease containing as an active ingredient the orally administered adsorbent described in any one of [1] to [6]; and

[8] A therapeutic or prophylactic agent for a hepatic disease containing as an active ingredient the orally administered adsorbent described in any one of [1] to [6]. Furthermore, the present specification discloses the following.

[9] A method of prophylaxis or therapy of a renal disease or a hepatic disease wherein the orally administered adsorbent described in any of [1] to [6] is administered in an effective dose to a renal disease or hepatic disease therapy subject;

[10] Surface-modified spherical activated carbon for use in (a) therapy (method) of a renal disease or a hepatic disease,

the surface-modified spherical activated carbon containing not less than 0.5 wt% nitrogen atoms, having specific surface area determined by the BET method from 800 $m^2/g$ to 3000 $m^2/g$, having an average particle size from 0.01 mm to 1 mm, and having total acidic group content of not less than 0.30 meq/g;

[11] The spherical activated carbon according to [10], wherein the average particle size of the surface-modified spherical activated carbon is from 50 μm to 200 μm;

[12] The spherical activated carbon according to [10] or [11], wherein the surface-modified spherical activated carbon has total acidic group content from 0.30 meq/g to 1.20 meq/g, and total basic group content from 0.20 meq/g to 1.20 meq/g;

[13] The orally administered adsorbent according to any one of [10] to [12], wherein the surface-modified spherical activated carbon is prepared using a thermoplastic resin, thermosetting resin, or ion exchange resin containing nitrogen atoms as a carbon source;

[14] The surface-modified spherical activated carbon according to [13], wherein the thermoplastic resin or ion exchange resin contains a monomer selected from the group consisting of acrylonitrile, ethylacrylonitrile, methylacrylonitrile, diphenylacrylonitrile, and chloroacrylonitrile;

[15] The surface-modified spherical activated carbon according to [13], wherein the thermosetting resin contains a monomer selected from the group consisting of melamine and urea;

[16] Use of surface-modified spherical activated carbon for production of a prophylactic or therapeutic medicine for a renal disease or a hepatic disease,

the surface-modified spherical activated carbon containing not less than 0.5 wt% nitrogen atoms, having specific surface area determined by the BET method from 800 $m^2/g$ to 3000 $m^2/g$, having an average particle size from 0.01 mm to 1 mm, and having total acidic group content of not less than 0.30 meq/g;

[17] The use of spherical activated carbon according to [16], wherein the average particle size of the surface-modified spherical activated carbon is from 50 $\mu$m to 200 $\mu$m;

[18] The use of surface-modified spherical activated carbon described in [16] or [17], wherein the surface-modified spherical activated carbon has total acidic group content from 0.30 meq/g to 1.20 meq/g, and total basic group content from 0.20 meq/g to 1.20 meq/g;

[19] The use of surface-modified spherical activated carbon described in any one of [16] to [18], wherein the surface-modified spherical activated carbon is prepared using a thermoplastic resin, thermosetting resin, or ion exchange resin containing nitrogen atoms as a carbon source;

[20] The use of surface-modified spherical activated carbon described in [19], wherein the thermoplastic resin or ion exchange resin contains a monomer selected from the group consisting of acrylonitrile, ethylacrylonitrile, methylacrylonitrile, diphenylacrylonitrile, and chloroacrylonitrile;

[21] The use of surface-modified spherical activated carbon described in [19], wherein the thermosetting resin contains a monomer selected from the group consisting of melamine and urea;

[22] Use of surface-modified spherical activated carbon for prophylaxis or therapy of a renal disease or a hepatic disease,

the surface-modified spherical activated carbon containing not less than 0.5 wt% nitrogen atoms, having specific surface area determined by the BET method from 800 $m^2$/g to 3000 $m^2$/g, having an average particle size from 0.01 mm to 1 mm, and having total acidic group content of not less than 0.30 meq/g;

[23] The use of spherical activated carbon described in [22], wherein the average particle size of the surface-modified spherical activated carbon is from 50 $\mu$m to 200 $\mu$m;

[24] The use of surface-modified spherical activated carbon described in [22] or [23], wherein the surface-modified spherical activated carbon has total acidic group content from 0.30 meq/g to 1.20 meq/g, and total basic group content from 0.20 meq/g to 1.20 meq/g;

[25] The use of surface-modified spherical activated carbon described in any one of [22] to [24], wherein the surface-modified spherical activated carbon is prepared using a thermoplastic resin, thermosetting resin, or ion exchange resin containing nitrogen atoms as a carbon source;

[26] The use of surface-modified spherical activated carbon described in [25], wherein the thermoplastic resin or ion exchange resin contains a monomer selected from the group consisting of acrylonitrile, ethylacrylonitrile, methylacrylonitrile, diphenylacrylonitrile, and chloroacrylonitrile;

[27] The use of surface-modified spherical activated carbon described in [25], wherein the thermosetting resin contains a monomer selected from the group consisting of melamine and urea.

Advantageous Effects of Invention

According to the orally administered adsorbent of the present invention, because adsorption ability for uremic substances, particularly adsorption ability for $\beta$-aminoisobutyric acid, is remarkably excellent, a large quantity of poisonous toxic substances can be adsorbed by a small quantity of orally administered adsorbent. Therefore, higher efficacy can be obtained by ingestion of the same quantity as a conventional orally administered adsorbent. Alternatively, the dosage for obtaining the same efficacy can be reduced from the dosage of a conventional orally administered adsorbent.

Brief Description of Drawings

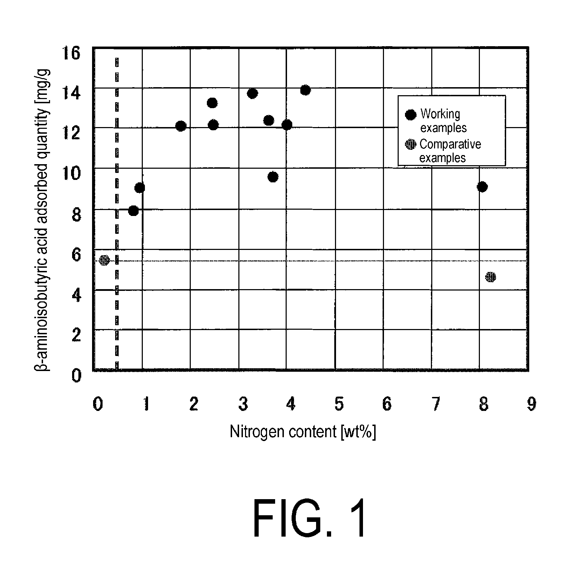

FIG. 1 is a graph showing the $\beta$-aminoisobutyric acid adsorbed quantity (24 hours) of orally administered adsorbents obtained in Working Examples 1 to 11 and Comparative Examples 1 and 2.

FIG 2 is a graph showing the relationship between nitrogen content and $\beta$-aminoisobutyric acid adsorbed quantity (24 hours) for spherical activated carbon of working examples having a BET specific surface area of approximately 1500 $m^2$/g (Working Examples 1, 2, 3, 4, and 8).

FIG. 3 is a graph showing the relationship between the BET specific surface area and $\beta$-aminoisobutyric acid adsorbed quantity (24 hours) of orally administered adsorbents obtained in Working Examples 1 to 11 and Comparative Examples 1 and 2.

FIG. 4 is a graph showing the relationship between an average particle size and $\beta$-aminoisobutyric acid adsorbed quantity (3 hours) for spherical activated carbon of working examples having a BET specific surface area of approximately 1300 $m^2$/g (Working Examples 8, 9, 10, and 11).

Description of Embodiments

[1] Orally administered adsorbent

Surface-modified spherical activated carbon used as the orally administered adsorbent according to the present invention means spherical activated carbon having acidic centers of not less than 0.30 meq/g. Conversely, surface-unmodified spherical activated carbon means spherical activated carbon having acidic centers of less than 0.30 meq/g. As described above, surface-modified spherical activated carbon is a porous body obtained by performing activation treatment after heat-treating a carbon precursor, and then further performing surface modification treatment by oxidation treatment and reduction treatment. It may exhibit appropriate degrees of interaction with acids and bases. On the other hand, surface-unmodified spherical activated carbon is a porous body obtained by performing activation treatment after heat-treating a carbon precursor, for example. It may be spherical activated carbon which has not subsequently undergone surface modification treatment by oxidation treatment and reduction treatment, or it may be spherical activated carbon obtained by performing heat treatment in a non-oxidative atmosphere after the aforementioned activation treatment.

The surface-modified spherical activated carbon used in the orally administered adsorbent of the present invention contains not less than 0.5 wt% nitrogen atoms, has specific surface area determined by the BET method from 800 $m^2$/g to 3000 $m^2$/g, has an average particle size from 0.01 mm to 1 mm, has total acidic group content from 0.30 meq/g to 1.20 meq/g, and has total basic group content from 0.20 meq/g to 1.20 meq/g.

(Nitrogen atom quantity)

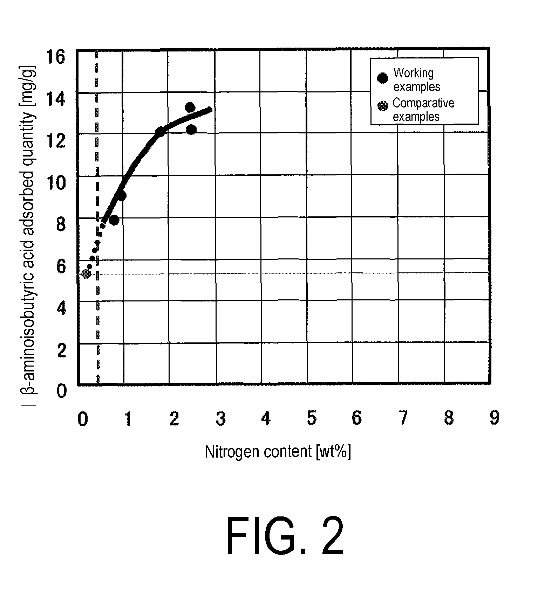

The nitrogen atom content of the surface-modified spherical activated carbon is not less than 0.5 wt%, preferably not less than 0.7 wt%, more preferably not less than 0.9 wt%, even more preferably not less than 0.95 wt%, and even more preferably not less than 1.0 wt%. When the nitrogen atom content is not less than 0.5 wt%, the rise in adsorption ability for uremic substances is remarkable, which is desirable. The upper limit of nitrogen atom content is not particularly limited, but not greater than 20 wt% is preferred. When the nitrogen atom content is not less than 0.5 wt%, the β-aminoisobutyric acid adsorbed quantity increases as nitrogen content increases. The β-aminoisobutyric acid adsorbed quantity is also influenced by a specific surface area. FIG. 2 illustrates the relationship between nitrogen content and β-aminoisobutyric acid adsorbed quantity (24 hours) for spherical activated carbon having a BET specific surface area of approximately 1500 $m^2$/g (Working Examples 1, 2, 3, 4, and 8). As is clear from FIG. 2, as the nitrogen content increased, the β-aminoisobutyric acid adsorbed quantity increased. In particular, when the nitrogen content was from 0.5 wt% to 3 wt%, a striking correlation between nitrogen content and β-aminoisobutyric acid adsorbed quantity was seen.

(Carbon source)

The carbon source of the surface-modified spherical activated carbon is not particularly limited provided that it contains nitrogen atoms, but examples include heat-fusible resins and heat-infusible resins.

(Heat-fusible resin)

Examples of heat-fusible resins include thermoplastic resins containing nitrogen atoms produced using a monomer containing nitrogen atoms (for example, crosslinked vinyl resin containing nitrogen atoms).
Examples of monomers containing nitrogen atoms for producing crosslinked vinyl resin containing nitrogen atoms include acrylonitrile, methylacrylonitrile (for example, 2-methylacrylonitrile), ethylacrylonitrile (for example, 2-hydroxyethylacrylonitrile, 2-(1-hydroxyethyl)acrylonitrile, 2-(2-fluoroethyl)acrylonitrile), diphenylacrylonitrile (for example, 2,3-diphenylacrylonitrile, 3,3-diphenylacrylonitrile), and chloroacrylonitrile (for example, 2-chloroacrylonitrile). A vinyl resin of a polymer of only these monomers containing nitrogen atoms, or a crosslinked vinyl resin of a copolymer with other monomers may be used.

The above crosslinked vinyl resins used as a carbon source may be a spherical polymer obtained by emulsion polymerization, bulk polymerization, or solution polymerization, or, preferably, a spherical polymer obtained by suspension polymerization. To make the spherical crosslinked vinyl resin uniformly infusible, it is necessary to form pores in the crosslinked vinyl resin in advance. Pores may be formed in the resin by adding a porogen at the time of polymerization. The BET specific surface area of the crosslinked vinyl resin required to make the crosslinked vinyl resin infusible is preferably not less than 5 $m^2$/g, and more preferably not less than 10 $m^2$/g.
For example, when preparing a crosslinked vinyl resin by suspension polymerization, a spherical crosslinked vinyl resin may be prepared by adding an organic phase containing a vinyl-based monomer, a crosslinking agent, a porogen, and a polymerization initiator to an aqueous dispersion medium containing a dispersion stabilizer, and after forming numerous organic droplets suspended in an aqueous phase by agitating to mix, and heating them to polymerize the monomer in the organic droplets.

As other monomers that form a copolymer with the above monomer containing nitrogen atoms, any vinyl-based monomer that can be formed into spheres can be used, examples of which include aromatic vinyl-based monomers such as styrene and styrene derivatives in which a vinyl group hydrogen or phenyl group hydrogen is substituted, and compounds in which a heterocyclic or polycyclic compound is bonded to a vinyl group instead of a phenyl group. More specific examples of aromatic vinyl-based monomers include $\alpha$- or $\beta$-methylstyrene, $\alpha$- or $\beta$-ethylstyrene, methoxystyrene, phenylstyrene, chlorostyrene, and the like, and o-, m-, or p-methylstyrene, ethylstyrene, methoxystyrene, methylsilylstyrene, hydroxystyrene, chlorostyrene, cyanostyrene, nitrostyrene, aminostyrene, and carboxystyrene, and sulfoxystyrene, sodium styrene sulfonate, and the like, and vinylpyridine, vinylthiophene, vinylpyrrolidone, vinylnaphthalene, vinylanthracene, vinylbiphenyl, and the like. Aliphatic vinyl-based monomers may also be used, specific examples of which include vinyl esters such as ethylene, propylene, isobutylene, diisobutylene, vinyl chloride, acrylic acid ester, methacrylic acid ester, vinyl acetate, and the like, vinyl ketones such as vinyl methyl ketone, vinyl ethyl ketone, and the like, vinyl aldehydes such as acrolein, methacrolein, and the like, and vinyl ethers such as vinyl methyl ether, vinyl ethyl ether, and the like. A crosslinked vinyl resin with a monomer containing nitrogen atoms may be prepared using one or more of these vinyl-based monomers, but methylstyrenes ($\alpha$-methylstyrene, $\beta$-methylstyrene, o-methylstyrene, m-methylstyrene, and p-methylstyrene), ethylstyrenes ($\alpha$-ethylstyrene and $\beta$-ethylstyrene), and styrene are preferred.

As the crosslinking agent, any crosslinking agent that can be used to crosslink the aforementioned vinyl-based monomers may be used, examples of which include divinylbenzene, divinylpyridine, divinyltoluene, divinylnaphthalene, diallyl phthalate, ethylene glycol diacrylate, ethylene glycol dimethylate, divinylxylene, divinylethylbenzene, divinylsulfone, and polyvinyl or polyallyl ethers of glycol or glycerol, polyvinyl or polyallyl ethers of pentaerythritol, polyvinyl or polyallyl ethers of mono or dithio derivatives of glycol, and polyvinyl or polyallyl ethers of resorcinol, and divinyl ketone, divinyl sulfide, allyl acrylate, diallyl maleate, diallyl fumarate, diallyl succinate, diallyl carbonate, diallyl malonate, diallyl oxalate, diallyl adipate, diallyl sebacate, triallyl tricarballylate, triallyl aconitate, triallyl citrate, triallyl phosphate, N,N'-methylenediacrylamide, 1,2-di($\alpha$-methylmethylenesulfonamide)ethylene, trivinylbenzene, trivinylnaphthalene, polyvinylanthracene, and trivinylcyclohexane. Particularly preferred crosslinking agents include polyvinyl aromatic hydrocarbons (for example, divinylbenzene), glycol trimethacrylates (for example, ethylene glycol dimethacrylate), and polyvinyl hydrocarbons (for example, trivinylcyclohexane). Divinylbenzene is most preferred due to its excellent pyrolysis characteristics.

Examples of suitable porogens include alkanols having from 4 to 10 carbons (for example, n-butanol, sec-butanol, 2-ethylhexanol, decanol, and 4-methyl-2-pentanol), alkyl esters having at least 7 carbons (for example, n-hexyl acetate, 2-ethylhexyl acetate, methyl oleate, dibutyl sebacate, dibutyl adipate, and dibutyl carbonate), alkyl ketones having from 4 to 10 carbons (for example, dibutyl ketone and methyl isopropyl ketone), alkyl carboxylates (for example, heptanoic acid), aromatic hydrocarbons (for example, toluene, xylene, and benzene), higher saturated aliphatic hydrocarbons (for example, hexane, heptane, and isooctane), and cyclic aliphatic hydrocarbons (for example, cyclohexane).

The polymerization initiator is not particularly limited, and one that is generally used in this field may be used, but an oil-soluble polymerization initiator that is soluble in the polymerizable monomer is preferred. Examples of the polymerization initiator include dialkyl peroxides, diacyl peroxides, peroxyesters, peroxydicarbonates, and azo compounds. More specific examples include dialkyl peroxides such as methyl ethyl peroxide, di-t-butyl peroxide, and dicumyl peroxide; diacyl peroxides such as isobutyl peroxide, benzoyl peroxide, 2,4-dicyclobenzoyl peroxide, and 3,5,5-trimethylhexanoyl peroxide; peroxyesters such as t-butyl peroxypivalate, t-hexyl peroxypivalate, t-butyl peroxyneodecanoate, t-hexyl peroxyneodecanoate, 1-cyclohexyl-1-methylethyl peroxyneodecanoate, 1,1,3,3-tetramethylbutyl peroxyneodecanoate, cumyl peroxyneodecanoate, and ($\alpha,\alpha$-bis-neodecanoylperoxy)diisopropylbenzene; peroxydicarbonates such as bis(4-t-butylcyclohexyl)peroxydicarbonate, di-n-propyl-oxydicarbonate, diisopropyl peroxydicarbonate, di(2-ethylethylperoxy)dicarbonate, dimethoxybutyl peroxydicarbonate, and di(3-methyl-3-methoxybutylperoxy)dicarbonate; and azo compounds such as 2,2'-azobisisobutyronitrile, 2,2'-azobis(4-methoxy-2,4-dimethylvaleronitrile), 2,2'-azobis(2,4-dimethylvaleronitrile), and 1,1'-azobis(1-cyclohexanecarbonitrile); and the like.

(Heat-infusible resin)

The heat-infusible resin used in the present invention is not limited provided that it contains nitrogen atoms, but specific examples include thermosetting resins containing nitrogen atoms (for example, melamine resin and phenol resin) and ion exchange resins containing nitrogen atoms.

(Melamine resin)

Melamine resin is a thermosetting resin categorized as an amino resin, and is produced by polycondensation of melamine and formaldehyde. Specifically, the starting material used is methylol melamine obtained by condensing melamine and formaldehyde under alkaline conditions. Heating the methylol melamine causes polycondensation, resulting in a thermosetting resin crosslinked in a mesh pattern.

Furthermore, melamine resin alone may be used as the melamine resin. Additionally, a resin of a copolymer of melamine resin with urea or phenol may be used.

(Urea resin)

Urea resin is produced by polycondensation of urea and formaldehyde. Specifically, urea and formaldehyde undergo a dehydration condensation reaction under alkaline conditions or acidic conditions to obtain a condensate. Urea resin alone may also be used as the urea resin. Additionally, a resin of a copolymer of urea resin with polyurethane, melamine resin, phenol, or the like may be used.

(Ion exchange resin containing nitrogen atoms)

The ion exchange resin is not limited provided that it contains nitrogen atoms, but an ion exchange resin having a structure in which an ion exchange group is bonded to a copolymer matrix having a three-dimensional mesh skeleton of crosslinked vinyl resin containing nitrogen atoms may be used. Depending on the ion exchange group, ion exchange resins are broadly classified into strongly acidic ion exchange resins having a sulfonic acid group, weakly acidic ion exchange resins having a carboxylic acid group or sulfonic acid group, strongly basic ion exchange resins having a quaternary ammonium salt, and weakly basic ion exchange resins having a primary or tertiary amine. Other special resins include so-called hybrid ion exchange resins having ion exchange groups of both an acid and a base. In the present invention, all of these ion exchange resins containing nitrogen atoms may be used as a carbon source.

(Diameter)

The diameter of the surface-modified spherical activated carbon used for the orally administered adsorbent according to the present invention is not particularly limited, but is preferably from 0.005 mm to 1.5 mm, more preferably from 0.01 mm to 1 mm, and even more preferably from 0.02 mm to 0.8 mm. When the diameter of the surface-modified spherical activated carbon is less than 0.005 mm, the exterior surface area of the surface-modified spherical activated carbon increases and adsorption of beneficial substances such as digestive enzymes readily occurs, which is undesirable. When the diameter exceeds 1.5 mm, the diffusion length of toxic substances into the surface-modified spherical activated carbon increases and the adsorption rate decreases, which is undesirable.

(Average particle size)

The particle diameter at a cumulative particle size percentage of 50% on a volume standard cumulative particle size distribution curve created using a laser diffraction-style particle size distribution analyzer is used as the average particle size (Dv50).

The range of average particle size of the surface-modified spherical activated carbon used for the orally administered adsorbent according to the present invention is not particularly limited provided that the average particle size is from 0.01 mm to 1 mm (from 10 $\mu$m to 1000 $\mu$m). When the average particle size of the surface-modified spherical activated carbon is less than 0.01 mm, the exterior surface area of the surface-modified spherical activated carbon increases and adsorption of beneficial substances such as digestive enzymes readily occurs, which is undesirable. When the average particle size exceeds 1 mm, the diffusion length of toxic substances into the surface-modified spherical activated carbon increases and the adsorption rate decreases, which is undesirable. The average particle size is preferably from 20 $\mu$m to 800 $\mu$m, and more preferably from 30 $\mu$m to 500 $\mu$m. In particular, surface-modified spherical activated carbon having an average particle size from 50 $\mu$m to 200 $\mu$m is most preferred because it has excellent initial adsorption ability, and in the general residence time in the upper small intestine, it can very rapidly adsorb poisonous toxic substances (particularly $\beta$-aminoisobutyric acid) in the body.

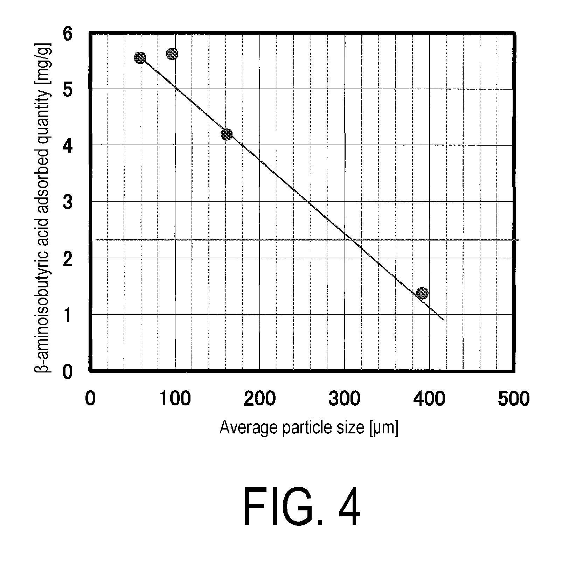

FIG. 4 illustrates the relationship between an average particle size and $\beta$-aminoisobutyric acid adsorbed quantity (3 hours) for spherical activated carbon having a BET specific surface area of approximately 1300 $m^2$/g (Working Examples 8, 9, 10, and 11). As is clear from FIG. 4, when an average particle size was from 50 $\mu$m to 200 $\mu$m, $\beta$-aminoisobutyric acid adsorbed quantity (3 hours) increased. Specifically, when the average particle size is from 50 $\mu$m to 200 $\mu$m, the initial adsorption ability in the body is excellent, which is desirable.

(Specific surface area)

The specific surface area of the surface-modified spherical activated carbon can be determined by the BET method or the Langmuir method. As the specific surface area of the surface-modified spherical activated carbon used for the orally administered adsorbent according to the present invention, the specific surface area determined by the BET method (sometimes abbreviated as "SSA" hereinafter) is from 800 m$^2$/g to 3000 m$^2$/g. With surface-modified spherical activated carbon having SSA less than 800 m$^2$/g, toxic substance adsorption performance decreases, which is undesirable. The lower limit of SSA is more preferably not less than 1000 m$^2$/g. The upper limit of SSA is not particularly limited, but from the perspective of strength, SSA is preferably not greater than 3000 m$^2$/g.

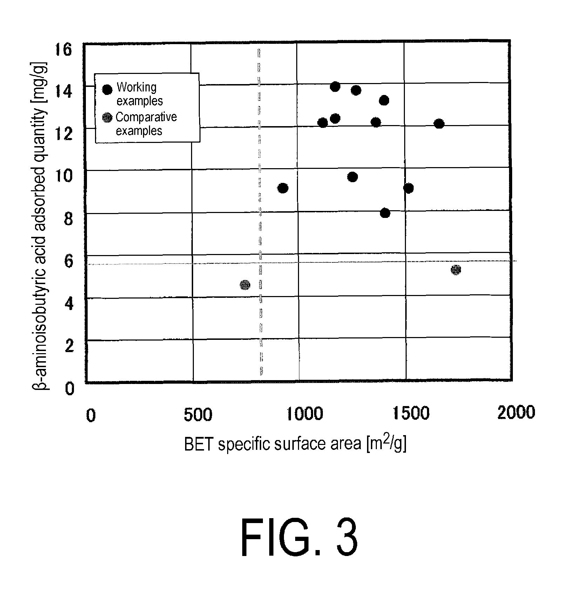

FIG. 3 illustrates the relationship between a BET specific surface area and β-aminoisobutyric acid adsorbed quantity. As is understood from FIG. 3, when BET specific surface area is less than 800 m$^2$/g, β-aminoisobutyric acid adsorbed quantity decreases even if nitrogen content is not less than 0.5 wt%, which is undesirable.

(Total acidic group content and total basic group content)

In the surface-modified spherical activated carbon used for the orally administered adsorbent according to the present invention, in the configuration of functional groups, total acidic group content is from 0.30 meq/g to 1.20 meq/g, and total basic group content is from 0.20 meq/g to 1.20 meq/g. A surface-modified spherical activated carbon that satisfies the conditions of total acidic group content from 0.30 meq/g to 1.20 meq/g and total basic group content from 0.20 meq/g to 1.20 meq/g in the configuration of functional groups has high adsorption performance of water-soluble toxins such as DL-β-aminoisobutyric acid, but has even higher DL-β-aminoisobutyric acid adsorption ability due to containing not less than 0.5 wt% nitrogen atoms. In functional group configuration, it is preferred that the total acidic group content is from 0.30 meq/g to 1.00 meq/g. The lower limit of total basic groups is preferably 0.30 meq/g and the upper limit is preferably 1.10 meq/g, while 1.00 meq/g is more preferred, and 0.90 meq/g is even more preferred.

(Surface modification)

The surface-modified spherical activated carbon used in the present invention can be obtained by performing oxidation treatment alone or oxidation treatment and reduction treatment on the surface-unmodified spherical activated carbon obtained using the above heat-fusible resin or heat-infusible resin as a carbon source. The oxidation treatment can be performed in an atmosphere containing from 0.1 vol% to 50 vol% oxygen, preferably from 1 vol% to 30 vol%, and particularly preferably from 3 vol% to 20 vol%, at a temperature from 300°C to 800°C, and preferably from 320°C to 600°C. The reduction treatment can be performed at a temperature from 800°C to 1200°C and preferably from 800°C to 1000°C in a non-oxidative gas atmosphere. In the specified oxygen-containing atmosphere, pure oxygen, nitrogen oxide, air, or the like may be used as the oxygen source. Furthermore, an atmosphere that is inert relative to carbon means nitrogen, argon, helium, or the like alone or in a mixture thereof. In this specification, surface-modified spherical activated carbon means a porous body obtained by performing the above oxidation treatment alone or oxidation treatment and reduction treatment on the above spherical activated carbon. By performing the oxidation treatment and reduction treatment, adsorption characteristics for toxic substances in the upper small intestine are improved due to the fact that an acidic point and a basic point are added in a good balance on the surface of the spherical activated carbon. For example, by performing oxidation treatment and reduction treatment on the above spherical activated carbon, specificity for toxic substances to be adsorbed can be improved.

Furthermore, the surface-modified spherical activated carbon produced in Working Example 12 is surface-modified spherical activated carbon that has undergone oxidation treatment alone and has not undergone reduction treatment. The surface-modified spherical activated carbon of Working Example 12 has superior β-aminoisobutyric acid adsorption quantity (24 hours or 3 hours) compared to a surface-modified spherical activated carbon that does not contain nitrogen atoms and has undergone only oxidation treatment.

(Pore volume)

The pore volume of pores having a pore diameter from 20 nm to 15,000 nm of the spherical activated carbon used in the orally administered adsorbent of the present invention is not particularly limited, but is preferably not greater than 1.00 mL/g, and more preferably not greater than 0.80 mL/g. The lower limit is not particularly limited, but is preferably not less than 0.01 mL/g.

The pore volume of pores having a pore diameter from 7.5 nm to 15,000 nm of the spherical activated carbon used in the orally administered adsorbent of the present invention is not particularly limited, but is preferably not greater than 1.00 mL/g, and more preferably not greater than 0.80 mL/g. The lower limit is not particularly limited, but is preferably not less than 0.01 mL/g.

The pore volume is measured using the mercury penetration method.

(Method for producing surface-modified spherical activated carbon)

When a heat-fusible resin (for example, crosslinked vinyl resin) is used as the carbon source, the spheres formed of heat-fusible resin soften and deform into a non-spherical shape or fuse to each other when heat-treated. Therefore, softening can be reduced by performing oxidation treatment in an atmosphere containing oxygen from 150°C to 400°C as infusibility treatment prior to the above activation treatment. Specifically, by so-called infusibility treatment such as oxidation treatment, the heat-fusible resin can be used in production of surface-modified spherical activated carbon after modifying the resin to a state in which melt oxidation can be avoided.

The crosslinked vinyl resin which is the heat-fusible resin softens and melts by the heat treatment in a non-oxidizing gas atmosphere and has a carbonization yield of less than 10%, but the crosslinked vinyl resin does not soften and melt by oxidation treatment in an atmosphere containing oxygen from 150°C to 400°C as infusibility treatment, and a spherical carbonaceous material can be obtained at a high carbonization yield of not less than 30%, and spherical activated carbon can be obtained by activation treatment on the resin in the same manner as the above heat-infusible resin.

In the preparation of the surface-modified spherical activated carbon used for the orally administered adsorbent of the present invention, when heat-infusible resin (for example, ion exchange resin) is used as a carbon source, substantially the same operations as conventional production methods using pitch can be employed. For example, spherical activated carbon can be obtained by first performing activation treatment under the flow of a gas that reacts with carbon (for example, steam or carbon dioxide gas) at a temperature from 700°C to 1000°C on spheres formed of heat-infusible resin. In a case where a large amount of pyrolysis gas is generated when the spheres of the heat-fusible resin after infusibility treatment or the heat-infusible resin are heat-treated, pyrolysis products can be removed in advance by appropriate pre-heat-treatment before the activation operation.

The above heat-infusible resin used as a starting material is a material that can be formed into spheres and, importantly, does not melt or soften and undergo shape deformation in heat treatment at a temperature not greater than 500°C.

For the above heat-infusible resin used as the starting material, it is desirable that carbonization yield by heat treatment is high. When carbonization yield is low, strength as surface-modified spherical activated carbon will be low. Since unnecessary pores are formed, the bulk density of the surface-modified spherical activated carbon is low and specific surface area per unit volume is low, and therefore the administered volume increases, which leads to the problem that oral administration is difficult. Therefore, a higher carbonization yield of the heat-infusible resin is preferred, and the value of yield by heat treatment in a non-oxidizing gas atmosphere at 800°C is preferably not less than 30 wt%, and more preferably not less than 35 wt%.

The surface-modified spherical activated carbon of the present invention can be obtained by performing oxidation treatment on the spherical activated carbon obtained by the above activation treatment in an atmosphere containing from 0.1 vol% to 50 vol% oxygen, preferably from 1 vol% to 30 vol%, and particularly preferably from 3 vol% to 20 vol%, at a temperature from 300°C to 800°C, and preferably from 320°C to 600°C, and then performing reduction treatment at a temperature from 800°C to 1200°C, and preferably from 800°C to 1000°C, in a non-oxidative gas atmosphere. In the specified oxygen-containing atmosphere, pure oxygen, nitrogen oxide, air, or the like may be used as the oxygen source. Furthermore, an atmosphere that is inert relative to carbon means nitrogen, argon, helium, or the like alone or in a mixture thereof. Here, surface-modified spherical activated carbon is a porous body obtained by performing oxidation treatment and reduction treatment on the above spherical activated carbon, and adsorption characteristics for toxic substances in the upper small intestine are improved due to that fact that acidic centers and basic centers are added in a good balance on the surface of the spherical activated carbon. For example, by performing oxidation treatment and reduction treatment on the above spherical activated carbon, specificity for toxic substances to be adsorbed can be improved.

(Control of physical properties of surface-modified spherical activated carbon)

When preparing the surface-modified spherical activated carbon according to the present invention using the above heat-fusible resin or heat-infusible resin, physical properties of the surface-modified spherical activated carbon (for example, average particle size, pore volume, specific surface area, and the like) can be controlled by various methods. For example, the average particle size of resin depends on the size of the droplets in the aqueous phase, and the size of the droplets can be controlled by the amount of suspending agent, the rotational frequency of stirring, the shape of the stirring blades, or the monomer ratio in the aqueous phase (ratio of amount of water to amount of monomer). For example, when the amount of suspending agent is increased, droplet size decreases, and when the rotational frequency of stirring is increased, droplet size decreases. Additionally, it is preferred that the amount of monomer in the aqueous phase is decreased from the perspective that not only coalescence of droplets can be controlled, but removal of heat of polymerization becomes easy as well. However, when the monomer ratio is too low, the amount of monomer per batch becomes small and the amount of synthetic resin obtained decreases, which is undesirable from the perspective of productivity.

Furthermore, when the pore diameter to be controlled is not less than 10 nm, the pore volume and specific surface area can be controlled primarily by the amount and type of porogen, and when the pore diameter to be controlled is not greater than 10 nm, the pore volume and specific surface area can be controlled by activation conditions using steam. For example, as the activation reaction, spherical activated carbon can be obtained by performing activation treatment under the flow of a gas that reacts with carbon (for example, steam or carbon dioxide gas) at a temperature from 700°C to 1000°C. The specific surface area can be controlled by the activation conditions. For example, the specific surface area can be increased by increasing the activation time, increasing the activation temperature, and increasing the concentration of the gas that reacts with carbon. Additionally, the fine structure as surface-modified spherical activated carbon can be controlled by the type of resin, the type and amount of crosslinking agent, the infusibility conditions, and/or the activation temperature.

[2] Orally administered adsorbent for therapy or prophylaxis of renal disease or hepatic disease

Because the surface-modified spherical activated carbon used for the orally administered adsorbent of the present invention has excellent adsorbance of hepatic disease aggravating substances and toxic substances in renal diseases, it may be used as an orally administered adsorbent for therapy or prophylaxis of a renal disease or may be used as an orally administered adsorbent for therapy or prophylaxis of a hepatic disease. Examples of the renal disease include chronic renal failure, acute renal failure, chronic pyelonephritis, acute pyelonephritis, chronic nephritis, acute nephritic syndrome, acute progressive nephritic syndrome, chronic nephritic syndrome, nephrotic syndrome, nephrosclerosis, interstitial nephritis, tubulopathy, lipoid nephrosis, diabetic nephropathy, renovascular hypertension, and hypertension syndrome, or secondary renal diseases attendant to these primary diseases. Another example is pre-dialysis mild renal failure, and it may be used for condition improvement of mild renal failure before dialysis or condition improvement during dialysis (see "Clinical Nephrology," Asakura Publishing, N. Honda, K. Koiso, K. Kurogawa, 1990 edition, and "Nephrology," Igaku Shoin, T. Onomae, S. Fujimi, editors, 1981 edition).

Examples of the hepatic disease include fulminant hepatitis, chronic hepatitis, viral hepatitis, alcoholic hepatitis, hepatic fibrosis, cirrhosis, hepatic cancer, autoimmune hepatitis, drug-induced allergic hepatitis, primary biliary cirrhosis, tremor, encephalopathy, metabolic disorder, and functional disorder. Otherwise, it may also be used in therapy of illnesses caused by harmful substances present in the body, that is, mental illness and the like.

Therefore, when the orally administered adsorbent according to the present invention is used as a therapeutic medicine for a renal disease, it contains the above surface-modified spherical activated carbon as an active ingredient. When the orally administered adsorbent of the present invention is used as a therapeutic medicine for a renal disease or a therapeutic medicine for a hepatic disease, the dosage thereof is influenced by whether the subject of administration is a human or other animal, and by age, individual differences, disease condition, or the like. Therefore, depending on the case, a dosage outside the following range may be appropriate, but in general, the orally administered dosage in humans is from 1 g to 20 g per day divided into three to four doses, and may be further adjusted according to symptoms. The administered form may be a powder, granules, tablet, sugar-coated pill, capsule, suspension, stick, individual package, emulsion, or the like. When ingested as a capsule, in addition to an ordinary gelatin capsule, an enteric-coated capsule may be used as necessary. When used as a tablet, it needs to be dissolved into microparticles in the body. Additionally, it may be used in the form of a complex blended with an electrolyte modifier such as alumigel or Kayexalate, which are other preparations.

Surface-modified spherical activated carbon containing not less than 0.5 wt% nitrogen atoms, having a specific surface area determined by the BET method from 800 $m^2$/g to 3000 $m^2$/g, having an average particle size from 0.01 mm to 1 mm, having total acidic group content from 0.30 meq/g to 1.20 meq/g, and having total basic group content from 0.20 meq/g to 1.20 meq/g can be used as a therapeutic or prophylactic agent for a renal disease or a therapeutic or prophylactic agent for a hepatic disease in the form of a mixture with conventional known surface-modified spherical activated carbon or surface-unmodified spherical activated carbon (that is, surface-modified or surface-unmodified spherical activated carbon containing less than 0.5 wt% nitrogen atoms).

Alternatively, surface-modified spherical activated carbon containing not less than 0.5 wt% nitrogen atoms, having a specific surface area determined by the BET method from 800 $m^2$/g to 3000 $m^2$/g, having an average particle size from 0.01 mm to 1 mm, having total acidic group content from 0.30 meq/g to 1.20 meq/g, and having total basic group content from 0.20 meq/g to 1.20 meq/g can be used as a therapeutic or prophylactic agent for a renal disease or a therapeutic or prophylactic agent for a hepatic disease by concomitant use with conventional known surface-modified spherical activated carbon or surface-unmodified spherical activated carbon (that is, surface-modified or surface-unmodified spherical activated carbon containing less than 0.5 wt% nitrogen atoms).

[3] Method of therapy of renal disease or hepatic disease

The surface-modified spherical activated carbon used in the orally administered adsorbent according to the present invention can be used in a method of prophylaxis or therapy of a renal disease or a hepatic disease. Therefore, the method of therapy of a renal disease or a hepatic disease of the present invention is characterized in that the above orally administered adsorbent containing surface-modified spherical activated carbon is administered in an effective dose to a renal disease or hepatic disease therapy subject.

The administration route, dosage, administration interval, and the like of the above surface-modified spherical activated carbon may be determined as appropriate in accordance with the type of illness, the age, gender, and body weight of the patient, the degree of symptoms, the dosing method, and the like.

[4] Surface-modified spherical activated carbon for use in a method of therapy of renal disease or hepatic disease

The surface-modified spherical activated carbon used in the orally administered adsorbent according to the present invention can be used in a method of prophylaxis or therapy of a renal disease or a hepatic disease. Therefore, the surface-modified spherical activated carbon of the present invention is for use in a method of prophylaxis or therapy of a renal disease or a hepatic disease.

The amount and the like of the above surface-modified spherical activated carbon used in prophylaxis or therapy may be determined as appropriate in accordance with the type of illness, the age, gender, and body weight of the patient, the degree of symptoms, the dosing method, and the like.

[5] Use of surface-modified spherical activated carbon for production of therapeutic medicine for renal disease or hepatic disease

The surface-modified spherical activated carbon used in the orally administered adsorbent according to the present invention can be used for producing a prophylactic or therapeutic medicine for a renal disease or a hepatic disease. Therefore, use of the present invention is use of surface-modified spherical activated carbon for producing a medicine for prophylaxis or therapy of a renal disease or a hepatic disease.

The contained amount and the like of the above surface-modified spherical activated carbon in the prophylactic or therapeutic medicine may be determined as appropriate in accordance with the type of illness, the age, gender, and body weight of the patient, the degree of symptoms, the dosing method, and the like.

[6] Use of surface-modified spherical activated carbon for therapy of renal disease or hepatic disease

The surface-modified spherical activated carbon used in the orally administered adsorbent according to the present invention can be used for therapy of a renal disease or a hepatic disease. Therefore, use of the present invention is use of surface-modified spherical activated carbon for prophylaxis or therapy of a renal disease or a hepatic disease.

The amount and the like of the above surface-modified spherical activated carbon used in prophylaxis or therapy may be determined as appropriate in accordance with the type of illness, the age, gender, and body weight of the patient, the degree of symptoms, the dosing method, and the like.

[Examples]

The present invention will be described in detail hereinafter using working examples, but these working examples do not limit the scope of the present invention.

Working Example 1

4500 g of deionized water, 0.9 g of sodium nitrite, and 6.8 g of Metalose 60SH-15 (Shin-Etsu Chemical Co., Ltd.) were put in a 10-L polymerization reactor. To this were added 376 g of styrene, 1049 of divinylbenzene (57% divinylbenzene and 43% ethylvinylbenzene), 75 g of acrylonitrile, 8.7 g of 2,2'-azobis(2,4-dimethylvaleronitrile), and 525 g of hexane as a porogen. The interior of the system was replaced with nitrogen gas, and this two-phase system was heated to 55°C while stirring at 180 rpm, and then held in that state for 20 hours. The obtained resin was washed with water and filtered, and then dried for 16 hours at 180°C under nitrogen flow, to produce a spherical porous synthetic resin having an average particle size of 197 $\mu$m.

The obtained spherical porous synthetic resin was put in a reaction tube with a grating, and infusibility treatment was performed in a vertical tube furnace. As the infusibility treatment, dry air was made to flow from bottom to top of the reaction tube, and after heating to 180°C, the temperature was raised from 180°C to 240°C in 3 hours, and then held at 240°C for 1 hour. The temperature was then raised from 240°C to 250°C in 30 minutes and held at 250°C for 2 hours, and then raised from 250°C to 260°C in 30 minutes and held at 260°C for 3 hours. The temperature was then raised from 260°C to 300°C in 2 hours and held at 300°C for 1 hour, and spherical porous oxidized resin was thereby obtained. The obtained spherical porous oxidized resin was heated in a nitrogen atmosphere at 850°C, and then, using a fluidized bed, activation treatment was performed in a nitrogen atmosphere containing steam at 850°C until the BET specific surface area reached 1440 $m^2$/g, and spherical activated carbon was thereby obtained. This was oxidation-treated for 3 hours at 470°C in an air atmosphere diluted with nitrogen using a fluidized bed. Then, it was reduction-treated for 17 minutes at 900°C in a nitrogen gas atmosphere using a fluidized bed, and surface-modified spherical activated carbon was obtained. Characteristics of the obtained surface-modified spherical activated carbon are shown in Table 1.

Working Example 2

Spherical porous synthetic resin was prepared by repeating the resin preparation operations of Working Example 1 except that 301 g of styrene and 150 g of acrylonitrile were used and the stirring rotational frequency of the two-phase system was 180 rpm. The average particle size of the obtained spherical porous synthetic resin was 193 $\mu$m. Surface-modified spherical activated carbon was prepared by repeating the infusibility treatment and activation treatment operations of Working Example 1 except that the above spherical porous synthetic resin was used, and activation treatment was performed until the BET specific surface area reached 1630 $m^2$/g. Characteristics of the obtained surface-modified spherical activated carbon are shown in Table 1.

Working Example 3

4500 g of deionized water, 6.0 g of sodium nitrite, and 6.8 g of Metalose 60SH-15 (Shin-Etsu Chemical Co., Ltd.) were put in a 10-L polymerization reactor. To this were added 582 g of styrene, 393 g of divinylbenzene (57% divinylbenzene and 43% ethylvinylbenzene), 525 g of acrylonitrile, 8.7 g of 2,2'-azobis(2,4-dimethylvaleronitrile), and 375 g of hexane as a porogen, and the interior of the system was then replaced with nitrogen gas. This two-phase system was heated to 55°C while stirring at 150 rpm, and then held in that state for 20 hours. The obtained resin was washed with water and filtered, and then dried for 16 hours at 180°C under nitrogen flow, to produce spherical porous synthetic resin having an average particle size of 171 $\mu$m.
The obtained spherical porous synthetic resin was put in a reaction tube with a grating, and infusibility treatment was performed in a vertical tube furnace. As the infusibility treatment, dry air was made to flow from bottom to top of the reaction tube, and after heating to 180°C, the temperature was raised from 180°C to 240°C in 3 hours, and then held at 240°C for 1 hour. The temperature was then raised from 240°C to 260°C in 1 hour and held at 260°C for 5 hours, and then raised from 260°C to 300°C in 2 hours and held at 300°C for 40 minutes, and spherical porous oxidized resin was thereby obtained. The obtained spherical porous oxidized resin was heated in a nitrogen atmosphere at 690°C, and then, using a fluidized bed, activation treatment was performed in a nitrogen atmosphere containing steam at 900°C until the BET specific surface area reached 1670 $m^2$/g, and spherical activated carbon was thereby obtained. This was oxidation-treated for 3 hours at 470°C in an air atmosphere diluted with nitrogen using a fluidized bed, and then reduction-treated for 17 minutes at 900°C in a nitrogen gas atmosphere using a fluidized bed, and surface-modified spherical activated carbon was obtained. Characteristics of the obtained surface-modified spherical activated carbon are shown in Table 1.

Working Example 4

Spherical porous synthetic resin was prepared by repeating the resin preparation operations of Working Example 3 except that 432 g of styrene and 675 g of acrylonitrile were used and the stirring rotational frequency of the two-phase system was 147 rpm. The average particle size of the obtained spherical porous synthetic resin was 190 $\mu$m. The obtained spherical porous synthetic resin was put in a reaction tube with a grating, and infusibility treatment was performed in a vertical tube furnace. As the infusibility treatment, dry air was made to flow from bottom to top of the reaction tube, and after heating to 180°C, the temperature was raised from 180°C to 240°C in 3 hours, and then held at 240°C for 1 hour. The temperature was then raised from 240°C to 260°C in 1 hour and held at 260°C for 5 hours, and spherical porous oxidized resin was thereby obtained. The obtained spherical porous oxidized resin was heated in a nitrogen atmosphere at 850°C, and then, using a fluidized bed, activation treatment was performed in a nitrogen atmosphere containing steam at 850°C until the BET specific surface area reached 1740 $m^2$/g, and spherical activated carbon was thereby obtained. This was oxidation-treated for 3 hours at 470°C in an air atmosphere diluted with nitrogen using a fluidized bed, and then reduction-treated for 17 minutes at 900°C in a nitrogen gas atmosphere using a fluidized bed, and surface-modified spherical activated carbon was obtained. Characteristics of the obtained surface-modified spherical activated carbon are shown in Table 1.

Working Example 5

Spherical porous synthetic resin was prepared by repeating the resin preparation operations of Working Example 4 except that 207 g of styrene, 900 g of acrylonitrile, and 450 g of hexane were used and the stirring rotational frequency of the two-phase system was 135 rpm. The average particle size of the obtained spherical porous synthetic resin was 172 $\mu$m.

Additionally, surface-modified spherical activated carbon was prepared by repeating the infusibility treatment and activation treatment operations of Working Example 4 except that the above spherical porous synthetic resin was used, and activation treatment was performed until the BET specific surface area reached 1280 m$^2$/g. Characteristics of the obtained surface-modified spherical activated carbon are shown in Table 1.

Working Example 6

Synthetic resin was prepared by repeating the resin preparation operations of Working Example 4 except that 1500 g of acrylonitrile, 0 g of styrene, and 0 g of divinylbenzene (57% divinylbenzene and 43% ethylvinylbenzene) were used and the stirring rotational frequency of the two-phase system was 140 rpm. The average particle size of the obtained spherical porous synthetic resin was 255 $\mu$m.

The obtained spherical porous synthetic resin was put in a reaction tube with a grating, and infusibility treatment was performed in a vertical tube furnace. As the infusibility treatment, dry air was made to flow from bottom to top of the reaction tube, and after heating to 180°C, the temperature was raised from 180°C to 240°C in 3 hours, and then held at 240°C for 1 hour. The temperature was then raised from 240°C to 260°C in 1 hour and held at 260°C for 4 hours, and spherical porous oxidized resin was thereby obtained. The obtained spherical porous oxidized resin was heated in a nitrogen atmosphere at 850°C, and then, using a fluidized bed, activation treatment was performed in a nitrogen atmosphere containing steam at 850°C until the BET specific surface area reached 1030 m$^2$/g, and spherical activated carbon was thereby obtained. This was oxidation-treated for 3 hours at 470°C in an air atmosphere diluted with nitrogen using a fluidized bed, and then reduction-treated for 17 minutes at 900°C in a nitrogen gas atmosphere using a fluidized bed, and surface-modified spherical activated carbon was obtained. Characteristics of the obtained surface-modified spherical activated carbon are shown in Table 1.

Working Example 7

Surface-modified spherical activated carbon was prepared by repeating the operations of Working Example 3 except that the activation temperature was 850°C instead of 900°C, and treatment was performed until the BET specific surface area reached 1080 m$^2$/g. Characteristics of the obtained surface-modified spherical activated carbon are shown in Table 1.

Working Example 8

Surface-modified spherical activated carbon was prepared by repeating the operations of Working Example 3 except that activation treatment was performed until BET specific surface area reached 1280 m$^2$/g. Characteristics of the obtained surface-modified spherical activated carbon are shown in Table 1.

Working Example 9

Spherical porous synthetic resin was prepared by repeating the resin preparation operations of Working Example 3 except that 13.5 g of Metalose 60SH-15 was used and the stirring rotational frequency of the two-phase system was 186 rpm. The average particle size of the obtained spherical porous synthetic resin was 135 $\mu$m.

Additionally, surface-modified spherical activated carbon was prepared by repeating the infusibility treatment and activation treatment operations of Working Example 2 except that the above spherical porous synthetic resin was used, and activation treatment was performed until the BET specific surface area reached 1200 m$^2$/g. Characteristics of the obtained surface-modified spherical activated carbon are shown in Table 1.

Working Example 10

Spherical porous synthetic resin was prepared by repeating the resin preparation operations of Working Example 3 except that 6.8 g of Metalose SM-400 (Shin-Etsu Chemical Co., Ltd.) was used instead of 6.8 g of Metalose 60SH-15 and the stirring rotational frequency of the two-phase system was 110 rpm. The average particle size of the obtained spherical porous synthetic resin was 367 $\mu$m.

The obtained spherical porous synthetic resin was put in a reaction tube with a grating, and infusibility treatment was performed in a vertical tube furnace. As the infusibility conditions, dry air was made to flow from bottom to top of the reaction tube, and after heating to 180°C, the temperature was raised from 180°C to 240°C in 3 hours, and then held at 240°C for 1 hour. The temperature was then raised from 240°C to 260°C in 1 hour and held at 260°C for 5 hours 40 minutes, and then raised from 260°C to 300°C in 2 hours and held at 300°C for 1 hour 30 minutes, and spherical porous oxidized resin was thereby obtained. The obtained spherical porous oxidized resin was heated in a nitrogen atmosphere at 850°C, and then, using a fluidized bed, activation treatment was performed in a nitrogen atmosphere containing steam at 850°C until the BET specific surface area reached 1280 $m^2$/g, and spherical activated carbon was thereby obtained. This was oxidation-treated for 3 hours at 470°C in an air atmosphere diluted with nitrogen using a fluidized bed, and then reduction-treated for 17 minutes at 900°C in a nitrogen gas atmosphere using a fluidized bed, and surface-modified spherical activated carbon was obtained. Characteristics of the obtained surface-modified spherical activated carbon are shown in Table 1.

Working Example 11

Spherical porous synthetic resin was prepared by repeating the resin preparation operations of Working Example 10 except that 3.4 g of SM-100 (Shin-Etsu Chemical Co., Ltd.) was used instead of 6.8 g of Metalose SM-400 and the stirring rotational frequency of the two-phase system was 75 rpm. The average particle size of the obtained spherical porous synthetic resin was 735 $\mu$m.

The obtained spherical porous synthetic resin was put in a reaction tube with a grating, and infusibility treatment was performed in a vertical tube furnace. As the infusibility conditions, dry air was made to flow from bottom to top of the reaction tube, and after heating to 180°C, the temperature was raised from 180°C to 240°C in 3 hours, and then held at 240°C for 1 hour. The temperature was then raised from 240°C to 260°C in 1 hour and held at 260°C for 5 hours 40 minutes, and then raised from 260°C to 300°C in 2 hours and held at 300°C for 1 hour, and spherical porous oxidized resin was thereby obtained. The obtained spherical porous oxidized resin was heated in a nitrogen atmosphere at 850°C, and then, using a fluidized bed, activation treatment was performed in a nitrogen atmosphere containing steam at 850°C until the BET specific surface area reached 1240 $m^2$/g, and spherical activated carbon was thereby obtained. This was oxidation-treated for 3 hours at 470°C in an air atmosphere diluted with nitrogen using a fluidized bed, and then reduction-treated for 17 minutes at 900°C in a nitrogen gas atmosphere using a fluidized bed, and surface-modified spherical activated carbon was obtained. Characteristics of the obtained surface-modified spherical activated carbon are shown in Table 1.

Comparative Example 1

4800 g of deionized water, 1.0 g of sodium nitrite, and 7.2 g of Metalose 60SH-15 (Shin-Etsu Chemical Co., Ltd.) were put in a 10-L polymerization reactor. To this were added 481 g of styrene, 1119 g of divinylbenzene (57% divinylbenzene and 43% ethylvinylbenzene), 9.3 g of 2,2'-azobis(2,4-dimethylvaleronitrile), and 560 g of hexane as a porogen, and the interior of the system was then replaced with nitrogen gas. This two-phase system was heated to 55°C while stirring at 140 rpm, and then held in that state for 20 hours. The obtained resin was washed with water and filtered, and then hexane was removed from the resin by evaporation by vacuum-drying. The resulting resin was vacuum-dried for 12 hours at 90°C, to obtain spherical porous synthetic resin having an average particle size of 246 $\mu$m.

The obtained spherical porous synthetic resin was put in a reaction tube with a grating, and infusibility treatment was performed in a vertical tube furnace. As the infusibility treatment, dry air was made to flow from bottom to top of the reaction tube, and after heating to 190°C, the temperature was raised from 190°C to 290°C at 10°C/minute, and spherical porous oxidized resin was thereby obtained. The obtained spherical porous oxidized resin was heated in a nitrogen atmosphere at 850°C, and then, using a fluidized bed, activation treatment was performed in a nitrogen atmosphere containing steam at 850°C until the BET specific surface area reached 1790 $m^2$/g, and spherical activated carbon was thereby obtained. This was oxidation-treated for 3 hours at 470°C in an air atmosphere diluted with nitrogen using a fluidized bed, and then reduction-treated for 17 minutes at 900°C in a nitrogen gas atmosphere using a fluidized bed, and surface-modified spherical activated carbon was obtained. Characteristics of the obtained surface-modified spherical activated carbon are shown in Table 1.

Comparative Example 2

Surface-modified spherical activated carbon was prepared by repeating the operations of Working Example 3 except that the heating temperature was 850°C instead of 690°C and activation treatment was not performed. Characteristics of the obtained surface-modified spherical activated carbon are shown in Table 1.

Comparative Example 3

Surface-modified spherical activated carbon was prepared by repeating the operations of Working Example 8 except that reduction treatment was not performed after oxidation treatment. Characteristics of the obtained surface-modified spherical activated carbon are shown in Table 1.

(Method for evaluating oral adsorbent)

The various characteristics shown in Table 1 below were measured by the following methods.

(1) Average particle size (Dv50)

The particle diameter at a cumulative particle size percentage of 50% on a volume standard cumulative particle size distribution curve created using a laser diffraction-style particle size distribution analyzer (SALAD-3000S; Shimadzu Corp.) was used as the average particle size (Dv50).

(2) Specific surface area (specific surface area calculation method by BET method)

The gas adsorption quantity of a spherical activated carbon sample can be measured using a specific surface area analyzer that uses the gas adsorption method (for example, ASAP2010 or AS6AP2020; Micromeritics Corp.), and a specific surface area can be calculated using the formula below. Specifically, a sample tube is packed with the spherical activated carbon sample, and after vacuum-drying at 350°C, post-drying sample weight is measured. Then, the sample tube is cooled to -196°C and nitrogen is introduced into the sample tube to adsorb nitrogen on the spherical activated carbon sample, and the relationship between nitrogen partial pressure and adsorbed quantity (adsorption isotherm) is measured.

A BET plot is created, with the relative pressure of nitrogen taken as p and the adsorbed quantity at that time taken as v ($cm^3$/g STP). Specifically, the range of p from 0.05 to 0.20 is plotted with $p/(v(1-p))$ on the vertical axis and p on the horizontal axis, and the specific surface area S (units: $m^2$/g) is determined by the following formula from the slope b (units: $g/cm^3$) and intercept c (units: $g/cm^3$) at that time.

[Formula 1]

$$S = \frac{MA \times \left(6.02 \times 10^{23}\right)}{22414 \times 10^{18} \times (b+c)}$$

MA is the cross-sectional area of a nitrogen molecule, and a value of 0.162 $nm^2$ was used here.

(3) Specific surface area (specific surface area calculation method by Langmuir equation)

The gas adsorption quantity of a spherical activated carbon sample can be measured using a specific surface area analyzer that uses the gas adsorption method (for example, ASAP2010 or ASAP2020; Micromeritics Corp.), and a specific surface area can be calculated using the Langmuir equation. Specifically, a sample tube is packed with the spherical activated carbon sample, and after vacuum-drying at 350°C, post-drying sample weight is measured. Then, the sample tube is cooled to -196°C and nitrogen is introduced into the sample tube to adsorb nitrogen on the spherical activated carbon sample, and the relationship between nitrogen partial pressure and adsorbed quantity (adsorption isotherm) is measured.

A Langmuir plot is created, with the relative pressure of nitrogen taken as p and the adsorbed quantity at that time taken as v ($cm^3$/g STP). Specifically, the range of p from 0.05 to 0.20 is plotted with p/v on the vertical axis and p on the horizontal axis, and the specific surface area S (units: $m^2$/g) is determined by the following formula when the slope at that time is taken as b (units: $g/cm^3$).

[Formula 2]

$$S = \frac{MA \times \left(6.02 \times 10^{23}\right)}{22414 \times 10^{18} \times b}$$

MA is the cross-sectional area of a nitrogen molecule, and a value of 0.162 nm$^2$ was used here.

(4) Elemental analysis (content of carbon, hydrogen, carbon, and oxygen atoms)

The organic elemental composition of a spherical activated carbon sample can be determined using an organic elemental analyzer (2400 Series II CHNS/O; PerkinElmer, Inc.). Specifically, 1.7 mg of sample was precisely weighed out and enclosed in a tin capsule. The sample was completely combusted in a 975°C combustion tube mounted on an organic elemental analyzer, and by measuring the amounts of carbon dioxide, water, and nitrogen dioxide in the produced gas, the contents (wt%) of carbon, hydrogen, and nitrogen atoms in the sample were determined. Furthermore, the oxygen content (wt%) was calculated by subtracting the total content (wt%) of carbon, hydrogen, and nitrogen atoms in the sample from 100 wt%.

(5) Pore volume by mercury penetration method

Pore volume can be measured using a mercury porosimeter (for example, Autopore 9200; Micromeritics Corp.). The spherical activated carbon sample is put in a sample container, and degassed for 30 minutes under pressure not greater than 2.67 Pa. Then, mercury is introduced into the sample container, pressure is gradually increased, and the mercury penetrates into the pores of the spherical activated carbon sample (maximum pressure: 414 MPa). From the relationship between pressure and mercury penetration quantity at this time, the pore volume distribution of the spherical activated carbon sample is measured using the calculation formulas below.

Specifically, the volume of mercury that penetrates the spherical activated carbon sample is measured from a pressure equivalent to pore diameter 21 $\mu$m (0.06 MPa) to the maximum pressure (414 MPa, equivalent to pore diameter 3 nm). In the calculation of pore diameter, when mercury penetrates into the pores of a cylinder having a diameter (D) at a pressure (P), and the surface tension of mercury is taken as "$\gamma$" and the contact angle between mercury and the pore wall is taken as "$\theta$," the following equation

$$-\pi D \gamma \cos\theta = \pi (D/2)^2 \cdot P$$

holds true based on the equilibrium of surface tension and the pressure that acts on the pore cross-section. Therefore,

$$D = (-4\gamma\cos\theta)/P$$

In this specification, the surface tension of mercury is taken as 484 dyne/cm and the contact angle between mercury and carbon is taken as 130 degrees. When the pressure P is expressed in MPa and the pore diameter D is expressed in $\mu$m, the relationship between the pressure P and the pore diameter D is determined using the following formula:

$$D = 1.24/P$$

For example, the pore volume in the range of pore diameter from 20 nm to 15,000 nm is equivalent to the volume of mercury that penetrates at mercury penetration pressure from 0.124 MPa to 165 MPa. Also, the pore volume in the range of pore diameter from 7.5 nm to 15,000 nm is equivalent to the volume of mercury that penetrates at mercury penetration pressure from 0.083 MPa to 165 MPa.

Furthermore, because the spherical activated carbon used for the orally administered adsorbent of the present invention has an extremely small particle size, the spaces between sample particles packed in the sample container are also small. Therefore, in the operation of pore volume measurement by the above mercury penetration method, there is a stage at which the mercury penetrates those interparticle spaces, and at that penetration stage, it behaves as if there are pores having a pore diameter from 8000 nm to 15,000 nm. It can be confirmed by observation using, for example, an electron microscope that no pores having a pore diameter from 8000 nm to 15,000 nm are present in the spherical activated carbon used for the orally administered adsorbent of the present invention. Therefore, in this specification, "pore volume in the range of pore diameter from 20 nm to 15,000 nm" and "pore volume in the range of pore diameter from 7.5 to 15,000 nm" also include the amount of mercury that penetrates the interparticle spaces.

(6) Total acidic group content

1 g of spherical activated carbon sample was added to 50 mL of 0.05 N NaOH solution, and this was shaken in a figure-eight with an amplitude of 3 cm at 76 cycles/minute for 48 hours using a figure-eight shaker (Triple Shaker NR-80; Taitec Corp.). The spherical activated carbon sample was then filtered out, and the consumed amount of NaOH determined by neutralization titration was taken as the total acidic group content.

(7) Total basic group content

1 g of spherical activated carbon sample was added to 50 mL of 0.05 N HCl solution, and this was shaken in a figure-eight with an amplitude of 3 cm at 76 cycles/minute at 37°C for 24 hours using a figure-eight shaker (Triple Shaker NR-80; Taitec Corp.). The surface-modified spherical activated carbon sample was then filtered out, and the consumed amount of HCl determined by neutralization titration was taken as the total basic group content.

(8) DL-β-aminoisobutyric acid adsorbed quantity test

After the spherical activated carbon sample was dried, 0.100 g of the dried sample was precisely weighed out and added to a 50-mL screw-cap vial containing 50 mL precisely weighted out of 1000 mL of liquid (stock solution) obtained by precisely weighing 0.100 g of β-aminoisobutyric acid in advance, adding phosphate buffer solution of pH 7.4 to the β-aminoisobutyric acid and dissolving the β-aminoisobutyric acid. The resulting solution was shaken for 3 hours or 24 hours at 37°C at 10 rpm using a mix rotor (Mix Rotor Variable VMR-5R; Asone Corp.). The contents in the screw-cap vial which had undergone shaking were suction-filtered using a membrane filter with 0.80 μm pores to produce a sample solution.
Meanwhile, as standard samples, 50 mL each of the stock solution, a mixture of the stock solution and phosphate buffer solution of pH 7.4 mixed in a ratio of 1:1, and phosphate buffer solution of pH 7.4 was put in a 50-mL screw-top vial, and was shaken for 3 hours or 24 hours at 37°C at 30 rpm using a mix rotor. The contents in the screw-cap vials which had undergone shaking were suction-filtered using a membrane filter with 0.80 μm pores to produce standard sample solutions.
For the sample solution and standard sample solutions, the organic carbon quantity was measured using a total organic carbon analyzer (TOC-L CPN; Shimadzu Corp.). A DL-β-aminoisobutyric acid calibration curve was created from the stoichiometric concentration of DL-β-aminoisobutyric acid versus organic carbon quantity in the standard sample solutions, and the DL-β-aminoisobutyric acid concentration Ct (mg/L) of the sample solution was determined using this curve.
The quantity of DL-β-aminoisobutyric acid adsorbed by the spherical activated carbon was determined using the following formula.

$$\text{DL-}\beta\text{-aminoisobutyric acid adsorbed quantity (mg/g)} = (C0 - Ct) \times V/Mt$$

Here, C0 is the DL-β-aminoisobutyric acid concentration (mg/L) of the stock solution, Ct is the DL-β-aminoisobutyric acid concentration (mg/L) of the sample solution, V is the initial volume of the sample solution (L), and Mt is the amount of spherical activated carbon (g).
The results are shown in Table 1.

[Table 1]

| | Average particle size [μm] | Specific surface area | | Elemental analysis | | | |
|---|---|---|---|---|---|---|---|
| | | Langmuir [m²/g] | BET [m²/g] | Carbon [wt%] | Hydrogen [wt%] | Nitrogen [wt%] | Oxygen [wt%] |
| Working Example 1 | 99 | 1940 | 1410 | 95.2 | 0.4 | 0.8 | 3.6 |
| Working Example 2 | 83 | 2100 | 1520 | 94.3 | 0.5 | 1.0 | 4.2 |
| Working Example 3 | 94 | 2270 | 1670 | 94.6 | 0.4 | 1.8 | 3.2 |
| Working Example 4 | 80 | 1850 | 1370 | 91.6 | 0.6 | 2.5 | 5.2 |
| Working Example 5 | 86 | 1620 | 1180 | 90.2 | 0.9 | 4.4 | 4.4 |
| Working Example 6 | 84 | 1280 | 930 | 85.3 | 1.2 | 8.1 | 5.5 |
| Working Example 7 | 77 | 1570 | 1120 | 90.8 | 0.9 | 4.0 | 4.0 |
| Working Example 8 | 97 | 1940 | 1410 | 93.8 | 0.3 | 2.5 | 3.4 |
| Working Example 9 | 66 | 1620 | 1180 | 91.4 | 0.8 | 3.6 | 4.2 |
| Working Example 10 | 161 | 1760 | 1280 | 91.5 | 0.8 | 3.3 | 4.4 |
| Working Example 11 | 392 | 1660 | 1260 | 90.4 | 0.8 | 3.7 | 5.1 |
| Comparative Example 1 | 110 | 2410 | 1740 | 95.0 | 0.3 | 0.3 | 4.5 |
| Comparative Example 2 | 86 | 990 | 750 | 85.9 | 1.2 | 8.2 | 4.7 |
| Comparative Example 3 | 106 | 1990 | 1450 | 80.9 | 1.1 | 2.0 | 16.0 |

[Table 1] (Continued)

| | Functional group quantity | | β-AIBA adsorbed quantity | | Pore volume | |
|---|---|---|---|---|---|---|
| | Total acidic group content [meq/g] | Total basic group content [meq/g] | 24 hours [mg/g] | 3 hours [mg/g] | 20-15,000 nm [mL/g] | 7.5-15000 nm [mL/g] |
| Working Example 1 | 0.44 | 0.55 | 7.87 | — | 0.05 | 0.11 |
| Working Example 2 | 0.45 | 0.58 | 9.02 | — | 0.11 | 0.27 |
| Working Example 3 | 0.53 | 0.62 | 12.06 | — | 0.08 | 0.10 |
| Working Example 4 | 0.38 | 0.72 | 12.14 | — | 0.29 | 0.39 |
| Working Example 5 | 0.31 | 0.77 | 13.86 | — | 0.53 | 0.55 |
| Working Example 6 | 0.31 | 0.77 | 9.06 | — | 0.52 | 0.53 |
| Working Example 7 | 0.30 | 0.76 | 12.14 | - | 0.08 | 0.09 |
| Working Example 8 | 0.48 | 0.67 | 13.19 | 5.62 | 0.16 | 0.17 |
| Working Example 9 | 0.32 | 0.74 | 12.34 | 5.55 | 0.15 | 0.16 |
| Working Example 10 | 0.31 | 0.75 | 13.66 | 4.19 | 0.04 | 0.07 |
| Working Example 11 | 0.34 | 0.76 | 9.55 | 1.38 | 0.03 | 0.12 |
| Comparative Example 1 | 0.52 | 0.38 | 5.31 | — | 0.04 | 0.08 |
| Comparative Example 2 | 0.32 | 0.74 | 4.56 | — | 0.07 | 0.08 |
| Comparative Example 3 | 1.94 | 0.03 | 3.25 | — | 0.11 | 0.22 |

**EP 2 959 908 A1**

Industrial Applicability

The orally administered adsorbent of the present invention may be used as an orally administered adsorbent for therapy or prophylaxis of a renal disease or may be used as an adsorbent for therapy or prophylaxis of a hepatic disease. Examples of the renal disease include chronic renal failure, acute renal failure, chronic pyelonephritis, acute pyelonephritis, chronic nephritis, acute nephritic syndrome, acute progressive nephritic syndrome, chronic nephritic syndrome, nephrotic syndrome, nephrosclerosis, interstitial nephritis, tubulopathy, lipoid nephrosis, diabetic nephropathy, renovascular hypertension, and hypertension syndrome, or secondary renal diseases attendant to these primary diseases. Another example is pre-dialysis mild renal failure, and the orally administered adsorbent may be used for condition improvement of mild renal failure before dialysis or condition improvement during dialysis (see "Clinical Nephrology," Asakura Publishing, N. Honda, K. Koiso, K. Kurogawa, 1990 edition, and "Nephrology," Igaku Shoin, T. Onomae, S. Fujimi, editors, 1981 edition).

Examples of the hepatic disease include fulminant hepatitis, chronic hepatitis, viral hepatitis, alcoholic hepatitis, hepatic fibrosis, cirrhosis, hepatic cancer, autoimmune hepatitis, drug-induced allergic hepatitis, primary biliary cirrhosis, tremor, encephalopathy, metabolic disorder, and functional disorder. Otherwise, the orally administered adsorbent of the present invention may also be used in therapy of illnesses caused by harmful substances present in the body, that is, mental illness and the like. The present invention has been described above using specific modes of embodiment, but modifications and improvements apparent to persons having ordinary skill in the art are also included in the scope of the present invention.

**Claims**

1. An orally administered adsorbent comprising surface-modified spherical activated carbon containing not less than 0.5 wt% nitrogen atoms, having a specific surface area determined by the Brunauer-Emmett-Teller (BET) method from 800 m$^2$/g to 3000 m$^2$/g, having an average particle size from 0.01 mm to 1 mm, and having total acidic group content of not less than 0.30 meq/g.

2. The orally administered adsorbent according to claim 1, wherein the average particle size of the surface-modified spherical activated carbon is from 50 $\mu$m to 200 $\mu$m.

3. The orally administered adsorbent according to claim 1 or 2, wherein the surface-modified spherical activated carbon has total acidic group content from 0.30 meq/g to 1.20 meq/g, and total basic group content from 0.20 meq/g to 1.20 meq/g.

4. The orally administered adsorbent according to any one of claims 1 to 3, wherein the surface-modified spherical activated carbon is prepared using a thermoplastic resin, thermosetting resin, or ion exchange resin containing nitrogen atoms as a carbon source.

5. The orally administered adsorbent according to claim 4, wherein the thermoplastic resin or ion exchange resin contains a monomer selected from the group consisting of acrylonitrile, ethylacrylonitrile, methylacrylonitrile, diphenylacrylonitrile, and chloroacrylonitrile.

6. The orally administered adsorbent according to claim 4, wherein the thermosetting resin contains a monomer selected from the group consisting of melamine and urea.

7. A therapeutic or prophylactic agent for a renal disease having as an active ingredient the orally administered adsorbent described in any one of claims 1 to 6.

8. A therapeutic or prophylactic agent for a hepatic disease having as an active ingredient the orally administered adsorbent described in any one of claims 1 to 6.

FIG. 1

FIG. 2

FIG. 3

FIG. 4

## INTERNATIONAL SEARCH REPORT

| | International application No. |
| --- | --- |
| | PCT/JP2014/054265 |

| A. CLASSIFICATION OF SUBJECT MATTER |
| --- |
| *A61K33/44*(2006.01)i, *A61K31/785*(2006.01)i, *A61P1/16*(2006.01)i, *A61P13/12*(2006.01)i, *A61P39/02*(2006.01)i |

According to International Patent Classification (IPC) or to both national classification and IPC

| B. FIELDS SEARCHED |
| --- |
| Minimum documentation searched (classification system followed by classification symbols) |
| A61K33/44, A61K31/785, A61P1/16, A61P13/12, A61P39/02 |

Documentation searched other than minimum documentation to the extent that such documents are included in the fields searched

| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
JSTPlus/JMEDPlus/JST7580(JDreamIII)

| C. DOCUMENTS CONSIDERED TO BE RELEVANT |
| --- |

| Category* | Citation of document, with indication, where appropriate, of the relevant passages | Relevant to claim No. |
| --- | --- | --- |
| Y | WO 2005/094845 A1 (Kureha Chemical Industry Co., Ltd.), 13 October 2005 (13.10.2005), claims 1 to 3; paragraphs [0026], [0027], [0041]; tables 1 to 4 & US 2008/0081073 A1 & EP 1745793 A1 | 1-8 |
| Y | JP 2004-168587 A (Toyota Central Research and Development Laboratories, Inc.), 17 June 2004 (17.06.2004), tables 2, 3 (Family: none) | 1-8 |
| Y | BOKI, K., Removal by Adsorption of Hydrogen Sulfide by a New Type of Activated Carbon Containing Nitrogen, Japanese Journal of Hygiene, 1983, Vol.83, No.5, p.877-882 | 1-8 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 07 April, 2014 (07.04.14) | 15 April, 2014 (15.04.14) |

| Name and mailing address of the ISA/ Japanese Patent Office | Authorized officer |
| --- | --- |
| Facsimile No. | Telephone No. |

Form PCT/ISA/210 (second sheet) (July 2009)

**INTERNATIONAL SEARCH REPORT**

International application No.

PCT/JP2014/054265

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2009-56449 A  (Kabushiki Kaisha Eiko), 19 March 2009 (19.03.2009), claims 1 to 3; examples 1 to 6; tables 1 to 6 (Family: none) | 1-8 |
| Y | JP 2009-269765 A  (The Kansai Coke and Chemicals Co., Ltd.), 19 November 2009 (19.11.2009), claims 1 to 5; paragraph [0014] (Family: none) | 4-8 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S6211611 B

- JP 2005314416 A

**Non-patent literature cited in the description**

- **N. HONDA ; K. KOISO ; K. KUROGAWA.** Clinical Nephrology. Asakura Publishing, 1990

- Nephrology. 1981